# EUROPEAN PATENT SPECIFICATION

(11) **EP 0 771 555 B1**
(45) Date of publication and mention of the grant of the patent: **14.11.2001**
(21) Application number: 95944700.4
(22) Date of filing: 03.08.1995
(51) Int. Cl.: A61K 7/00, A61K 7/48, A61K 38/46

(54) **AGENT FOR EXTERNAL APPLICATION WITH NUTRIENT AND PROTECTIVE PROPERTIES**
WIRKSTOFF ZUR ÄUSSERLICHEN ANWENDUNG MIT NÄHR- UND SCHUTZEIGENSCHAFTEN
AGENT A APPLICATION EXTERNEAUX PROPRIETES NUTRITIVES ET PROTECTRICES

(30) Priority: 03.08.1994 UA 94086487
(43) Date of publication of application: 07.05.1997
(73) Proprietor: Goudzenko, Janna Prokophievna, Kiev, 252167 (UA); Korotkaia, Elena Valerievna, Kiev, 252167 (UA)
(72) Inventor: Goudzenko, Janna Prokophievna, Kiev, 252167 (UA); Korotkaia, Elena Valerievna, Kiev, 252167 (UA)
(74) Representative: Grünecker, August, Dipl.-Ing.
(86) International application number: PCT/UA95/00005
(87) International publication number: WO 96/05797

(56) References cited:
- EP-A- 0 330 583
- EP-A- 0 378 936
- EP-A- 0 530 862
- WO-A-91/04010
- WO-A-93/10755
- WO-A-93/21899
- CH-A- 670 951
- DE-A- 3 514 724
- FR-A- 2 492 659
- FR-A- 2 521 005
- FR-A- 2 557 452
- SU-A- 1 782 590
- SU-A- 1 804 835
- US-A- 4 369 180
- DATABASE WPI Section Ch, Week 9545 Derwent Publications Ltd., London, GB; Class D21, AN 95-348299 XP002054031 & JP 07 238 010 A (KANEBO LTD) , 12 September 1995
- DATABASE WPI Section Ch, Week 8544 Derwent Publications Ltd., London, GB; Class D21, AN 85-272733 XP002054032 & JP 60 184 005 A (KANEBO LTD) , 19 September 1985

## Description

This invention relates to the medical field, exactly to cosmetology aiming to find new effective cosmetical remedies including also nutritional skin creams restoring skin physiological functions.

### PREVIOUS STATE OF ART IN THIS HELD:

Investigations concerning drugs research to assure the best skin nutritional functions are between the most important cosmetology problems,the skin metabolism being the main back ground of its functional activities and regeneration ability.

The system of humane skin integuments whose functions correlate with those ones of internal organs interacts continuously with environment. Being the interface between the environment and organism the human skin is always directly influenced by different environmental factors and variable parameters of organism inner system; so skin regulative mechanisms are always active and ready to induce systemic changes necessary to bring back, to normalize any prepared or realized pathological events concerning skin integument morphology and activities. A lot of trophic processes assuring the adequate consumption of increased affluence of energetical and plastic substances according to skin increased needs become guarantors of morphological and functional stability of skin structures. So the state of integuments determines the realization of metabolic processes necessary for skin cell viability and activity leading to she presence of healthy skin pecularities such as elasticity, turgor properties, humidity, pigmentation etc.

In the course of restoration process all the skin tissues use not only their own resources, but also these ones of the whole organism. To date, the dermocosmetology possesses different nutritional creams of different determined trophotropic and metabolotropic properties.

A well known cosmetical remedy containing equal quantities of lanoline, plant oil and water causes decreased heat emission after its application on the skin, the last becomes hotter; the increased blood flow causes skin teguments hyperemia activating skin metabolism followed by optimization of the skin functions (Yu.K.Skripkin, F.A.Zvier'kova, G.Ya.Sharapova, A.A.Studnitsyn. Guide Book on Children Dermatology. Moscow, Medicina Publishing House, 1983, 476 p.p., p.p.64-66, 227, 314; in Russian). Such an effect may be accelerated by biologicaly active substances included into the ointment - vitamines, enzymes, amino acids, lecithin, tissues preparations, phytoergogenic and other biologically active substances stimulating skin metabolism and nutrition (N.B.Koroliova. In the: "Guide Book of Medicinal Cosmetology", ed.V.T.Glukhen'ki, Kyiv, Zdorovia Publishing House, 1989, 302 p.p., p.p. 86-97; in Russian). For patients with dry deciduous skin of decreased turgor and elasticity accompanied by accelarated wrinkles formation and other indications suggesting precoce skin fading and aging nutritional vitamine-containing creams are usually applicated; they contain mostly vitamines A and E (T.V.Ptchiolkina, Ye.A.Sobolieva, I.G.Shishkina. In the: "Guide Book of Medicinal Cosmetology", Leningrad, Meditsina Publishing House, 1978, 176p.p., p.p.15-16).

However, this well known vitamines-containing external remedy does not assure a necessary result having no adequate effect on tissues perfusion and nutritional homeostasis.

The external remedy used in dermatology being the most relative in its action to our dermotherapeutic composition is the ointment containing salicylic acid (2.4 mass %), lanoline (24.4 mass %), vaseline (24.4 mass %), oil vitamine A solution (24.4 mass %), and water (24.4 mass %) (Guide Book of Medicinal Cosmetology, ed. A.F.Abakhadze, Moscow, Medicina Publishing House, 1975, 253 p.p., p.71). This remedy is aimed to abolish skin dryness, hardness, and rugosity caused by hyperkeratosis. Lanoline, vaseline and oil are used here as a fat ointment base, vitamine A and salicylic acid, as oxybenzoic acid derivative, being skin ameliorating active substances.

The defect of this remedy composition is its low effectivity due to:
- its acting factor being only a symptomes-directed one aimed to abolish only the consequences but not a cause of the pathological condition;
- factor of the substrate deficiency appearing due to the changed skin trophic homeostasis is not taken into account;
- the role of some biologically active substances causing skin cells damage is also not taken into consideration;
- the adequate skin trophic necessary for higher energetical processes level restoring damaged tissue structures cannot be achieved using such an ointment.

### DISCLOSURE OF THE INVENTION

The aim of this invention is to find an external skin remedy containing adequate combination of definite components assuring a set of physiological events; the tropho-metabolic activity of the external remedy increases due to adequate ingredients ratio causing a high trophoprotective effect followed by a stabile restoration of skin physiology functions suggesting this ointment high effectiveness.

Such an aim is possible to be solved because the external skin remedy proposed here containing a fat ointment base, an oxybenzoic acid derivate, vitamine A and water contains according to this invention salicylic acid or its derivates as an oxybenzoic acid derivate and in addition contains d-camphor, biogenic GABAergic substances, biogenic dopaminergic substances, M-cholinolytics, pancreatine, ascorbic acid, pantothenic acid calcium salt, vitamine D2, the mass % ratio of these compounds being the following:

| | |
|---|---|
| Fat ointment base | 30.0 - 70.0 |
| Vitamine A (retinol acetate) | 0.1 - 0.3 |
| Salicylic acid or its derivatives | 0.5 - 1.6 |
| d-Camphor | 2.0- 4.0 |
| Biogenic GABAergic substances | 0.8 - 1.5 |
| Biogenic dopaminergic substances | 0.8 - 1.5 |
| M-cholinolytics | 0.01- 0.1 |
| Pancreatine | 1.0 - 2.0 |
| Ascorbic acid | 0.5 - 1.0 |
| Pantothenic acid calcium salt | 0.5 - 2.5 |
| Vitamine D2 (ergocalciferol) | 0.000625-0.00125 |
| Water | all the rest |

The proposed composition of dermotropic substances containing some interacting components such as salicylic acid or its derivatives, d-camphor, biogenic neuromediators agents, M-cholinolytics, pancreatine and vitamines taken in adequate concentrations permits to increase trophic and biological activity of the proposed remedy composition and to obtain the highest trophoprotective effect. The synergism of the components asssures the adequate vascular circulation, corrects both energy and plastic deficiencies and restores trophic homeostasis followed by skin metabolism and physiology optimization. The complex of salicylic acid or its derivatives (mass % 0.5-1.6) with d-camphor (mass % 2.0-4.0) is included into this skin cosmetical ointment for microcirculation intensification as well as for better blood circulation in skin tissue, better perfusion and quick transport of physiologically active substances contained by the remedy proposed here.

Salicylic acid, a compound of high lipidotropism loosening readily epidermic tissues and possessing also anti-inflammatory effect, optimizes vascular circulation promoting cosmetical biostimulators transport into different skin layers. Some lipophilic and surface activity properties of d-camphor as well as its ability to penetrate into the skin and to normalize vascular tonicity interact with salicylic acid or its derivatives assuring not only better vascular circulation and perfusion but also increasing the penetrating ability of other medicative components contained in the ointment accelerating trophoprotective effect of external skin remedy. It becomes clinically evident because of skin integument becoming rose, fresh and smooth; the face looses its dropsicalence, the skin pigmentation becomes more regular, and some infiltrates disappear.
The use of lower per cent quantities both of salicylic acid or its derivatives and of d-camphor comparing to those indicated above as well as their ratio changes does not permit to restore microcirculation so effectively and quickly; the use of these substances in higher concentrations causes skin irritation due both to d-camphor irritative effect and salicylic acid keratolytic action.

The group of neurotropic substances including GABAergic ones, dopaminergic compounds and M-cholinolytics, their mass % being 0.8-1.5, 0.8-1.5, and 0.01-0.1, respectively, is given to the dermotological cosmetical ointment in order to correct neuromediators disbalance as well as substrate deficiency; these both phenomena are the background of skin trophic damage changing its physiological patterns. The importance of the mentioned neuromediators correction takes its way from the common brain and epidermis origin in the course of embryo development, some structures similar in their function and morphology being present in human brain and epidermis; there is a lot of histological data suggesting skin cells interaction with peripheral nerve fibrils.
Some tissue metabolites, biogenic substances possessing neuromediatory GABAergic and dopaminergic effect, are neurotransmitters of basal brain ganglia including also the highest vegetative nerval centers (reticular formation, hypothalamus); they influence the catecholamines level. In this regulation process, dopamin originated from L-DOPA of tissues has a trophic effect on neurons suggesting these substances to be used together. At the same time, the presence of L-DOPA and GABA in the external skin ointment permits to correct partly the substrat deficiency.
The simultaneous presence of M-cholinolytics (i.e. atropine sulfate, 0.01-0.1 mass %) blocking the cholinergic mediation of the vegetative nerval system permits to correct the physiologically active substances disbalance appearing in damaged tissues.
All these events favorize skin trophic optimization, normalization of metabolic processes, the reactions of physiological and reparative regeneration becoming more marked. They are usually followed by epitelization and melanogenesis process, marked edema having been abolished; the skin pigmentation becomes normal, its integuments rose and flesh-coloured; the tissue turgor increases; our patients become cheerful and are in better spirits.
It is impossible to obtain such results without use some additional biologically active substances - GABAergic and dopaminergic compounds as well as M-cholinolytics in the concentrations mentioned above. Any decreased content of these substances comparing to declarated ones leads to lower trophic and restorating effects. At the same time, the increased concentrations of GABAergic compounds, of dopaminergic ones, and of M-cholinolytics (above 1.5 mass %, 1.5 mass %, and 0.1 mass %, respectively) causes poorer restorating properties of the remedy; some patients feel even burning after such ointment application on the skin. The results of skin functional restoration due to neuromediative compounds action (GABAergic, dopaminergic substances and M-cholinolytics) become better after addition of such biogenic stimulators as pancreatin and vitamines mixture including vitamines A, D2, ascorbic acid, and pantothenic acid calcium salt.

Pancreatin, a preparation containing enzymes, is included into our ointment, its mass % being 1.0-2.0. This preparation being a dried animal pancreas tissue, its effect after application may be due not only to its enzymatical activity permitting, for instance, wound surface purification and scars resolving but also to its ability to control metabolic processes. So the pancreatin introduction into the external remedy of this invention combined with regulatory neurotropic GABAergic substances, dopaminergic ones and M-cholinolytics in the ratios indicated here permits to accelarate its trophic and metabolic activity.

The decrease of pancreatin content below 1 mass % reduces the effect of the external remedy of our invention and prolongates the beginning of trophic and restoring events; no increase of pancreatin above 2 mass % is accompanied by accelarated remedy effect, so 1-2 mass % of this component is usually introduced into the ointment.

The mixture of water-soluble vitamins (ascorbic acid and pantothenic acid calcium salt, 0.5-1.0 and 0.5-2.5 mass %, respectively) and oil-solubles ones (A and D2, 0.1-0.3 and 0.000625-0.00125 mass %, respectively) added to the external remedy accelerates its trophic effect.

It is also possible to add into the external remedy of our invention an antihistaminic preparation, dimedrole (0.05-0.1 mass %), as a blocking agent inhibiting tissue damage mediators; in such a way it is possible to increase the correction of the developed disbalance of physiologically active tissue substances.

The external trophoprotective remedy of our invention may also contain dimexide (1.0-3.0 mass %). This reagent able to penetrate through biological membranes is a good pilot of drug compounds helping other ointment components to go without any difficulty through skin integuments and accelerating their activities and synergism. It is especially important for patients with dystrophic skin phenomena and tissue perfusial damages.

Any dermocosmetical composition containing fats as well as natural and synthetic lipid-like substances (lanoline, spermaceti, cacao butter, bee wax, plant oils, vaseline etc.) may be suitable for external remedy of our invention, these substances mass % being 30-70; such an emulsified lipid system may be of "water-in-oil" or "oil-in- water" type.
In such a way, the marked trophoprotective properties of a given external skin remedy,and its metabolic activities are due to synergism of our adequately chosen drugs combination: neurotropic drugs (GABAergic and dopaminergic mediators, M-cholinergic neuroceptor inhibitors), enzymes-containing pancreatin preparation, vitamines mixture supplemented with lipotropic substances and dimexide increasing medicative components penetrating ability after skin application of the drug composition of our invention.

The external remedy of our invention may also contain any flavour compound (0.5-1.5 mass %) such as rose oil, lavender oil etc., favouring this remedy use as a dermocosmetical nutritional cream.

The external remedy of trophoprotective action is to be prepared as follows: d-Camphor is to be grinded with fat butter to obtain a homogenous camphor-butter mass; the last one is to be supplemented with vaseline (if it is added as fat ointment base) and with salicylic acid or its derivatives; they are to be mixed with fat butters; we continue then to grind the camphor-salicylo-butter mixture up to the obtaining of butter consistency (mixture 1).

All the components of the fat ointment base - non-liquid fats and lipid-like substances (water-free lanoline, spermaceti, bee wax, emulsive waxes et al.) are to be completely dissolved at water bath, the temperature being 70-75 C and then to be taken away from the bath.

Grinding continuously the fat ointment base obtained before we are to introduce consequently into it little portions of GABAergic substances (in water solution), pacreatin suspension in a water solution of pantothenic acid calcium salt, water solutions of M-cholinolytics, dopaminergic substances, ascorbic acid and also dimexide if necessary. In such a way it is possible to obtain an emulsified lipid complex containing in addition active biological substances (mixture 2). Both mixture, 1 and 2, are to be joint in the process of intensive mixing followed by oil vitamines solution and flavor addition. To assure the higher stability of emulsified system obtained, it is possible to use any adequate emulsifier. The dermocosmetical remedy of trophoprotective effect prepared in such a way is to be stored at +4-8° C in a glass container protecting from light.

### THE BEST WAY TO REALIZE THIS INVENTION

Elaborating this external remedy of trophoprotective effect we prepared its samples varying different components ratio and taking into consideration both physico-chemical parameters and well as therapeutico-preventive properties of the remedy. Some examples of remedy compositions prepared according to this invention are presented in the Table.

The components the most well know and the most widely used in the field of cosmetical dermatology were taken for these samples. So, lanoline, vaseline, seed oil, spermaceti, and bee wax were taken as fat ointment base; - aminobutyric acid was used as a GABAergic substance, L-DOPA as a dopaminergic one; atropin sulfate was used as a M-cholinolytic and lavender oil as a flavour agent.

All the samples of the external dermocosmetical remedy prepared according to prescriptions presented in the Table are of good consistency and colour; they possess high metabolic activity and trophoprotective effect; they penetrate well into skin tissues, regulate the skin metabolism and restore skin physiological functions. If compositions 1, 4, 7, and 10 are used, the organoleptic effect is found to be retarded comparing to this one obtained with other variants of this composition. At the same time, the use of samples 3, 6, 9, and 12 of the external dermocosmetical remedy caused in single cases a transient feeling of skin nteguments picking. The most optimal components ratios were shown to be contained in samples 2, 5, 8, and 11 causing the most favourable skin rophoprotective effect. However, taking into account some physico-chemical parameters of the external dermocosmetical remedy, its commodity properties and Organoleptic effect the variant 11 was found to be the best due to the quickest organoleptic results obtained with it concerning better cosmetical effects ncluding better skin pigmentation, increased tissues turgor and skin elasticity as well as remedy stability.

Below we describe some examples of use of our external dermocosmetical emedy of trophoprotective effect.

### Example 1.

A women, 45 years old. Her face is pale, puffy, with bags under her eyes, her skin being of sallow complexion, with hyperpigmentation loci on cheek-bones and lateral neck surfaces. Soft tissues are of decreased elasticity, the skin being dry, exhausted and flabby, especially on the lower part of face and on the neck. Some deep and surface wrinkles are seen near eyes angles, on the forhead and on lower eyelids; transversal neck wringles as well as fan-shaped upper lip wrinkles are also remarkable.

Cream-containing face masks are prescribed prepared according to the composition 4 (see Table).

The cream was applicated on the purified face and neck skin and kept during 30-40 min. The rest of the cream was then carefully taken away from the skin using a soft paper napkin. Such a procedure was made each day during a month, 30 procedures being necessary for a course of treatment.

During this treatment a general improvement of patient's appearence was detected accompanied by tissues elasticity increasing and surface wrinckles smoothing, the neck and face skin having become brighter in 10-12 days after the beginning of our dermocosmetic remedy use. The course of cream masks treatment having been finished, the skin became more elastic, surface wrinckles were smoothed, forhead and nose-bridge wrinckles were already less deep, puffy face appearence went away, bags under eyes were rather less. The face skin became rose, of regular colour pigmentation, having lost its local hyperpigmentation.

No side effects, skin irritation, and allergic reactions were found during treatment.

The effect obtained of skin physiological functions restoration was later observed to be stabile.

### Example 2.

A woman, 32 years old. The skin on her face, neck, and hands is dry and dehydrated, desquamation is seen on her forhead and chin. Sometimes, pruritis pesters also this patient. Tissue turgor is decreased, skin flaccidity is seen near eyes, on the forhead and nose-bridge part of face. Some wrinckles are seen in eyes angles and lower eyelids. Face skin pigmentation is irregular because of local hypo- and depigmentation on the chin and temporal regions as well as because of hyperpigmentation around the mouth, on temporal region, on the right part of forhead near the hairiness region of the head. Two years ago vitiligo was diagnosticated, so the patient treated by a dermatologist was put on a diet and had some inner trophotropic therapy courses.

Cream-containing masks were prescribed of the composition 9 given in the Table according to the mode of use described in the example 1, 25 masks having been prescribed for a course of treatment.

The good results of the treatment were seen in 5-7 days after its beginning, the better tissue turgor having been observed; the skin became "alive", elastic, less dry, of better appearence, the contrast between hyper- and depigmented loci becoming rather less, mostly because of hyperpigmentation zones becoming brighter. The treatment course having been finished, the skin was already elastic and smooth accompanied with the pruritis disappearence. The wrinckles near eye angles were smoothed, those ones on lower eyelids became less remarkable. A tendency was detected of skin pigmentation improvement; it become more regular both because of hyperpigmented loci brightening and bright flesh-coloured loci appearence in depigmentation regions.

During several first days of treatment using the composition 9 (see Table) the patient had the feeling of skin integuments picking.

### Example 3.

A woman, 52 years old. Her skin is dry, flaccid, very thin. Tissues turgor is flabby and accompanied by upper eyelids and lower ones deformation (prolapsus and bags under eyes, respectively). Deep and surface wrinckles of forhead, eyes angles, and neck; hirsutism of the upper lip. Diffused face skin hyperpigmentation causing sallow skin complexion.

Cream-containing masks of trophoprotective effect (composition 11 given in the Table) were used according to the mode of application described in the example 1, 28 masks having been applicated during the course of treatment.

The organoleptic effect of the treatment was already detected rather quickly - in 7-8 days after the treatment beginning; marked turgor improvement, smoothing of surface wrinckles, better skin integument pigmentation getting from day to day roser made the face more fresh. The treatment course having been finished, tissue turgor became satisfactory, skin was elastic, not dry, the bags under eyes disappeared. Some deep wrinckles became less remarkable, the surface ones of eye angles were smoothed. The patient's face became fresh, with the skin more bright, rose, with decreased upper lip hirsutism.

No unpleasant feelings and side effects were recorded.

The effect of skin restoration was suggested to be stabile during our further observations.

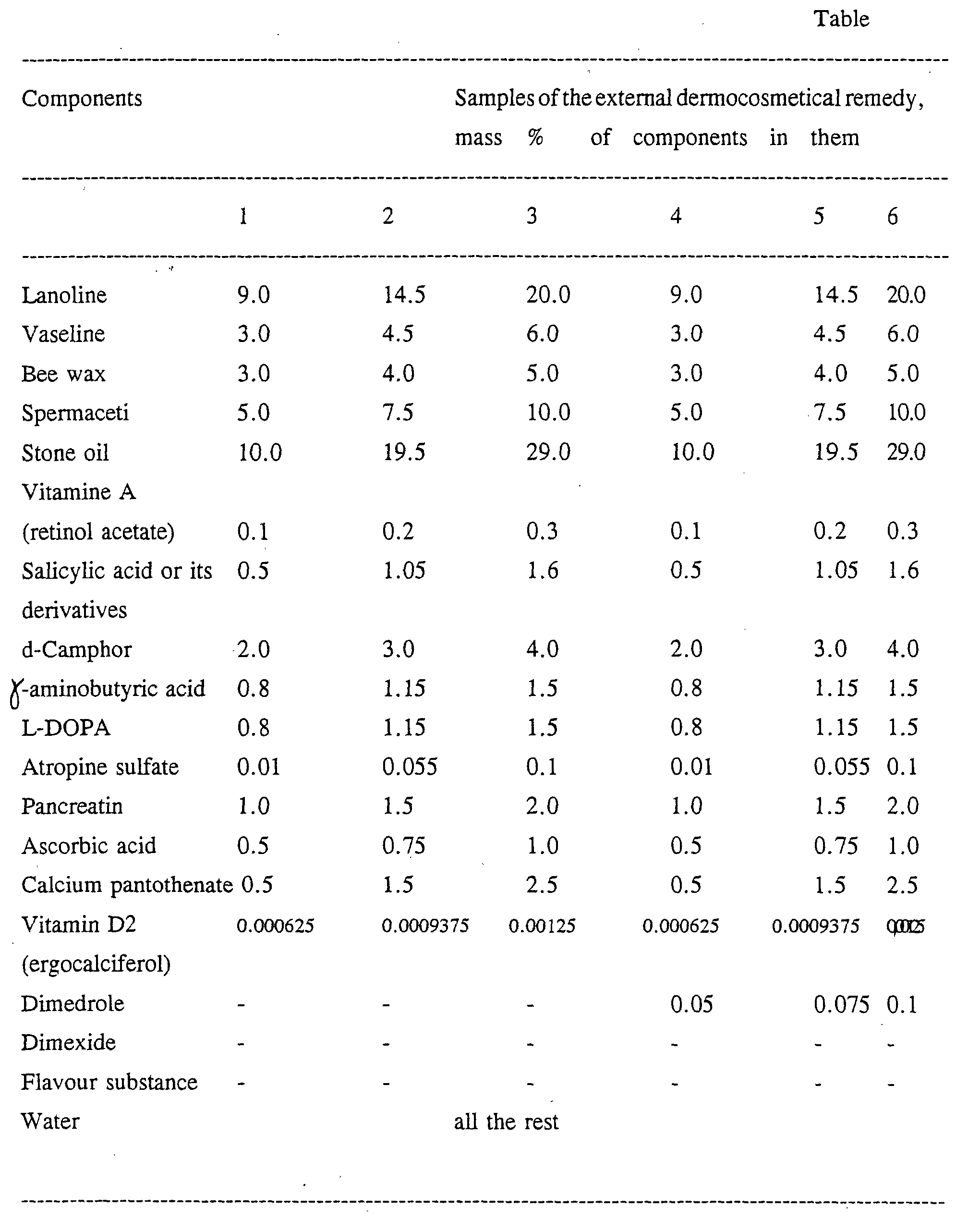

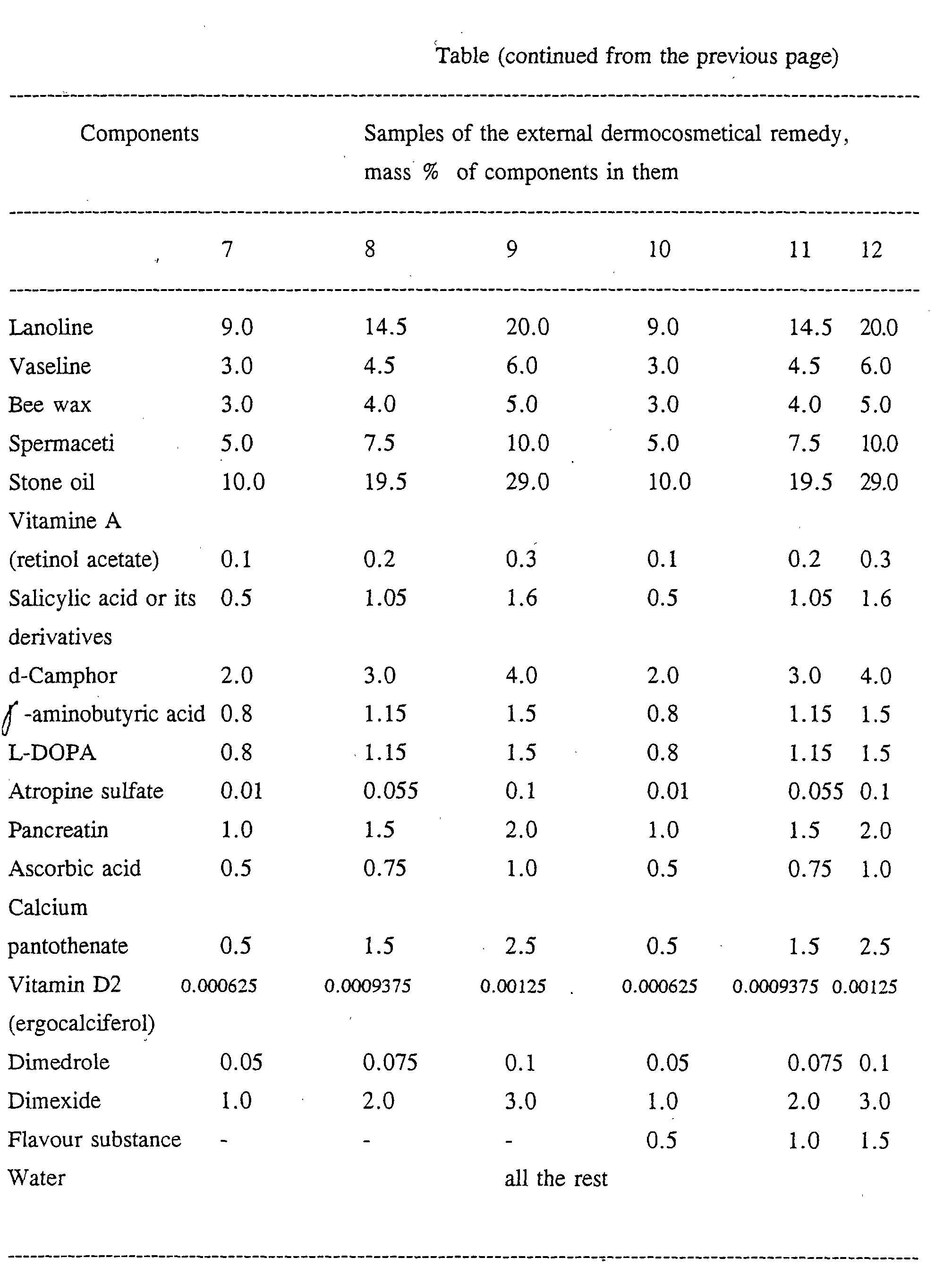

### RESULTS OF CLINICAL AND EXPERIMENTAL INVESTIGATIONS

Clinical investigations of the givenremedy were made with 120 patients (18-60 years old) having skin fading as well as very thin desquamated skin with spots, wrinckles, and damaged pigmentation. The results of investigations show that the nutritional remedy makes softer the normal, dry and flaccid skin inhibiting its aging. After treatment course using nutritional masks the skin becomes less dry, the pruritis, any feeling of stiffness and edema disappear, the skin becomes rose, smooth, elastic, of high turgor, with smoothed little wrinckles and more regular pigmentation. The positive results of the given dermocosmetical remedy use for skin applications and its therapeutic and preventive properties are detectable in 5-7 days after the beginning of treatment; later they become more pronounced, the favorable effect of the remedy becoming wider and more evident; the treatment course during 25-30 days of masks application assures stabile therapeutic effect.

The measuring of the skin temperature during cream-containing masks application shows it to become 2-3 degrees higher comparing to the temperature at the beginning of the mask treatment. The data concerning tissues electric resistance prove also the better circulation of treated tissues.

The given external remedy of trophoprotecting effect causes no unpleasant feelings as well as no skin irritation or allergic phenomena; no negative effect on skin morphological structures were found.

The electron microscopic investigations of volonteers skin bioptates suggest the presence of intracellular metabolism activation such as increased quantity of active mitochondria and abundance of cytoskeleton elements. Some histochemical data concerning the increase of succinate dehydrogenase activity due to oxidation-reduction processes prove indirectly the intensification of energetical skin metabolism.

Detailed investigations concerning posible toxic properties of our dermocosmetical remedy preceded further clinical studies; such investigations were made both in chronic and acute experiments with rats and white mice; our results prove the remedy investigated to be practically harmless, to possess no allergic properties and to demonstrate no negative effect on skin cells morphology and other organs of experimental animals.

And what is more - our data (of organoleptic, histochemical and histological studies) prove the pronounced ability of the remedy to optimize cells function and to restore normal skin physiology. So, we observed the increased growth of animals hair; in histological sections the basal epidermic layer was seen containing epitheliocytes with round "fulfilled" nuclei rich in chromatin grains; in a lot of hair follicles both in upper and lower parts of the external hair root sheath epithelial cells were found containing basophilic cytoplasm and large "fulfilled" nuclei, this fact proving indirectly increased cell function. In the basal-cell layer of skin there was no coarsened interstitial tissue; instead of it, tender collagen and reticular fibrils were seen with round cell elements among them represented by fibroblasts, macrophages and other mononuclears as well as a lot of moderate blooded vessels. Our histochemical studies show increased succinate dehydrogenase activity in rat basal epidermis and skin adnexa suggesting indirectly the higher level of energetical processes.

### INDUSTRIAL USE

The elaborated external remedy of trophoprotective effect is of high activity and permits to achieve good results concerning stabile restoration of skin functions and physiology becoming of full value. The remedy may be succesfully used as a dermocosmetical one to abolish different skin deficiencies due to its trophic and metabolism disorders: skin aging and fading making it thin, dry, hard, rigid, desquamative, of decreased turgor and elasticity. The use of the remedy described here accompanied by internal trophotropic drugs brings also positive results in cases of pigmentation disorders due to vitiligo and melanoderma development.

## Claims

1. Composition for the external remedy of trophoprotective action, containing fat ointment base, salicylic acid or ist derivative, vitamine A, water, d-camphor, biogenic GABAergic substances, biogenic dopaminergic substances, M-cholinolytics, pancreatin, ascorbic acid, calcium pantothenate, vitamin D2, wherein the components are present in the following ratio (in mass %):
| | |
|---|---|
| Fat ointment base | 30.0 - 70.0 |
| Vitamine A (retinol acetate) | 0.1 - 0.3 |
| Salicylic acid or its derivatives | 0.5-1.6 |
| d-Camphor | 2.0-4.0 |
| Biogenic GABAergic substances | 0.8 - 1.5 |
| Biogenic dopaminergic substances | 0.8-1.5 |
| M-cholinolytics | 0.01 - 0.1 |
| Pancreatine | 1.0-2.0 |
| Ascorbic acid | 0.5-1.0 |
| Pantothenic acid calcium salt | 0.5 - 2.5 |
| Vitamine D2 (ergocalciferol) | 0.000625 - 0.00125 |
| Water | all the rest |

2. The composition according to claim 1, containing γ aminobutyric acid (GABA) as the GABAergic substance in a quantity of 0.8-1.5 mass %, L-DOPA as the dopaminergic substance in a quantity of 0.8-1.5 mass %, and atropin sulfate as M-choliinolytic in a quantity of 0.01-0.1 mass %.

3. The composition according to claim 1, containing further dimedrole, an antihistaminic agent, as a tissue damage mediator bloker in a quantity of 0.05-0.1 mass %.

4. The composition according to claim 1 or 3, further containing dimexide in a quantity of 1.0-3.0 mass %.

5. The composition according to claim 1, 3 or 4, further containing a flavor substance in a quantity of 0.5-1.5 mass %.

6. Use of the composition according to any of claims 1 to 5 for the manufacture of a preparation for a dermocosmetological treatment.

## Patentansprüche

1. Zusammensetzung für ein äußerliches Arzneimittel mit einer trophoprotektiven Wirkung, enthaltend eine Fettsalbengrundlage, Salicylsäure oder ihr Derivat, Vitamin A, Wasser, d-Campher, biogene GABAerge Substanzen, biogene dopaminerge Substanzen, M-Cholinolytika, Pankreatin, Ascorbinsäure, Calciumpantothenat, Vitamin D2,
wobei die Komponenten in dem folgenden Verhältnis (in Masse-%) vorhanden sind:
| | |
|---|---|
| Fettsalbengrundlage | 30,0 - 70,0 |
| Vitamin A (Retinolacetat) | 0,1 - 0,3 |
| Salicylsäure oder ihre Derivate | 0,5- 1,6 |
| d-Campher | 2,0-4,0 |
| biogene GABAerge Substanzen | 0,8 - 1,5 |
| biogene dopaminerge Substanzen | 0,8 - 1,5 |
| M-Cholinolytika | 0,01-0,1 |
| Pankreatin | 1,0-2,0 |
| Ascorbinsäure | 0,5- 1,0 |
| Pantothensäure-Calciumsalz | 0,5- 2,5 |
| Vitamin D2 (Ergocalciferol) | 0,000625 - 0,00125 |
| Wasser | der gesamte Rest |

2. Zusammensetzung nach Anspruch 1, enthaltend γ-Aminobuttersäure (GABA) als GABAerge Substanz in einer Menge von 0,8 - 1,5 Masse-%, L-DOPA als dopaminerge Substanz in einer Menge von 0,8 - 1,5 Masse-% und Atropinsulfat als M-Cholinolytikum in einer Menge von 0,01 - 0,1 Masse-%.

3. Zusammensetzung nach Anspruch 1, außerdem enthaltend Dimedrol, ein Antihistaminikum, als Gewebeschaden-Mediator-Blocker in einer Menge von 0,05 - 0,1 Masse-%.

4. Zusammensetzung nach Anspruch 1 oder 3, außerdem enthaltend Dimexid in einer Menge von 1,0 - 3,0 Masse-%.

5. Zusammensetzung nach Anspruch 1, 3 oder 4, außerdem enthaltend einen Aromastoff in einer Menge von 0,5 - 1,5 Masse-%.

6. Verwendung der Zusammensetzung nach einem der Ansprüche 1 bis 5 für die Herstellung einer Zubereitung für eine dermatokosmetische Behandlung.

## Revendications

1. La composition pour le reméde extérieur d'action trophoprotecteur, contenant l'onguent gros à la base, l'acide salicylique ou son dérivé, la vitamine A, l'eau, d-camphre, les substances biogéniques GABAérgiques, les substances biogéniques dopaminérgiques, M-cholinolytiques, la pancréatine, l'acide ascorbique, la calcium pantothénate, la vitamine D2, **caractérisée en ce que** les composantes sont présent dans la proportion suivante (en masse %):
| | |
|---|---|
| Base de l'onguent gros | 30.0-70.0 |
| Vitamine A (retinol acétate) | 0.1-0.3 |
| Acide salicylique ou ses dérivés | 0.5-1.6 |
| d-Camphre | 2.0 - 4.0 |
| Substances biogéniques GABAérgiques | 0.8 - 1.5 |
| Substances biogéniques dopaminérgiques | 0.8 - 1.5 |
| M-cholinolytiques | 0.01-0.1 |
| Pancréatine | 1.0-2.0 |
| Acide ascorbique | 0.5-1.0 |
| Sel de calcium d'acide pantothénique | 0.5 - 2.5 |
| Vitamine D2 (ergocalciferol) | 0.000625 - 0.00125 |
| Eau | Tout le reste |

2. La composition selon la revendication 1, contenant γ-l'acide aminobutyrique (GABA) comme la substance GABAérgiques en quantité de 0.8-1.5 masse %, L-DOPA comme la substance dopaminérgiques en quantité de 0.8-1.5 masse %, et le sulfate d'atropine comme M-cholinolytique en quantité de 0.01 - 0.1 masse %.

3. La composition selon la revendication 1, contenant en outre le dimedrole, un agent antihistaminique comme le bloquer médiator du dommage du tissu en quantité de 0.05-0.1 masse%.

4. La composition selon les revendications 1 ou 3, contenant en outre le dimexide en quantité de 1.0-3.0 masse %.

5. La composition selon les revendications 1 ou 3 ou 4, contenant en outre la substance parfumée en quantité de 0.5-1.5 masse %.

6. Utilisation de la composition selon l'une des revendications 1 à 5 pour la manufacture de la préparation pour le traitement dermocosmétologique.
